# EUROPEAN PATENT SPECIFICATION

(11) **EP 3 801 279 B1**
(45) Date of publication and mention of the grant of the patent: **12.07.2023**
(21) Application number: 19726685.1
(22) Date of filing: 27.05.2019
(51) Int. Cl.: A61B 18/18, A61B 18/00, A61B 8/08, A61N 7/02, A61N 7/00, G16H 50/30

(54) **APPARATUS AND METHOD FOR ESTIMATING A LEVEL OF THERMAL ABLATION**
VORRICHTUNG UND VERFAHREN ZUR SCHÄTZUNG DES NIVEAUS EINER THERMISCHEN ABLATION
APPAREIL ET PROCÉDÉ D'ESTIMATION D'UN NIVEAU D'ABLATION THERMIQUE

(30) Priority: 29.05.2018 WO PCT/CN2018/088842
(43) Date of publication of application: 14.04.2021
(73) Proprietor: Koninklijke Philips N.V., 5656 AG Eindhoven (NL)
(72) Inventor: LI, Junbo, 5656 AE Eindhoven (NL); CHEN, Yinan, 5656 AE Eindhoven (NL); HUANG, Lingyun, 5656 AE Eindhoven (NL); LI, Fan, 5656 AE Eindhoven (NL); CHEN, Jiangang, 5656 AE Eindhoven (NL); LI, Xiaomin, 5656 AE Eindhoven (NL)
(74) Representative: Philips Intellectual Property & Standards
(86) International application number: PCT/EP2019/063565
(87) International publication number: WO 2019/228942

(56) References cited:
- US-A1- 2015 272 653
- US-A1- 2016 015 417

## Description

### FIELD OF THE INVENTION

The present invention relates to evaluate thermal ablation of tissue, and more particularly to an apparatus and method for estimating a level of thermal ablation using ultrasound echo data.

### BACKGROUND OF THE INVENTION

Thermal treatment or ablation uses heat induced, for example by means of the radiofrequency, microwave or HIFU (high intensity focused ultrasound), to ablate a target tissue region, such as a target lesion. It is minimal invasive, has shorter recovery time and less complications, can be used repeatedly and in conjunction with other treatment options. It has been widely used for the treatment of liver cancer, kidney cancer, lung cancer and heart rhythm disturbance, etc. It is important to monitor the procedure of the thermal ablation.

There are several types of existing technologies for thermal ablation monitoring, including: (1) using echogenicity change in ultrasound image, where the echogenicity change (i.e. bright cavitation bubble cloud caused by thermal ablation) has been used in clinical practice as the rough estimation of the ablation location; (2) using contrast enhanced CT or MRI; (3) using thermal couple or thermal resistance to detect the temperature change; (4) using Magnetic Resonance Thermometry to obtain accurate, real-time tissue temperature map; (5) using ultrasound thermometry to measure temperature; (6) using ultrasound elastography to measure tissue strain. US2016015417A1 disclosed a system for thermal treatment or ablation of tissue. The system is configured to measure ultrasound echo strain to estimate heat-induced structural changes of tissue, and further configured to compensate for motion artifacts when calculating the ultrasound echo strain. The motion artifacts may arise from the slippage of the tissue sample or the positional error of a mechanical 3D ultrasound probe. The underline assumption of ultrasound elastography based ablation monitoring is that the ablated region will turn stiffer due to tissue necrosis. However, tissue may soften during ablation and then stiffen after cooling down. Furthermore, after ablation, there might be soft edema region surrounding ablated zone. All those complicated tissue intrinsic stiffness change will result in difficulty for stiffness map interpretation.
US2015/272653A1 disclosed using multiple different types of parameters a input and/or a machine-learnt classifier to detect the completion of treatment from a tissue alternation perspective based on the transition that makes temperature estimation less reliable.

### SUMMARY OF THE INVENTION

It would be advantageous to provide an improved approach for estimating a level of thermal ablation of a tissue (also referred to as a level of tissue ablation) using ultrasound echo data.

It is known that the thermal ablation will make the tissue stiffer, and so it has been proposed to using ultrasound elastography, for example in US2016015417A1, to estimate the level of thermal ablation. The inventors have firstly recognized that before the ablation, the tissue to be ablated (e.g. lesion) normally stick to the surrounding tissues due to the microvascular which connect to and feed the lesion, and during the ablation, the tissue will become less sticky to the surrounding tissue due to cell necrosis upon thermal ablation (e.g. microvascular will be destroyed), resulting in more out-of-plane motion. On basis of such observation or recognition, the inventors have proposed to utilize the relationship between the level of thermal ablation and the out-of-plane motion to estimate the level of tissue treatment or ablation.

In accordance with an embodiment of a first aspect of the present invention, there is proposed an apparatus for estimating a level of thermal ablation for a tissue region on basis of ultrasound echo data of the tissue region. The apparatus comprises a data interface configured to receive the three-dimensional ultrasound echo data of the tissue region; and a data processor configured to measure in-plane strain and out-of-plane motion regarding the tissue region on basis of the received ultrasound echo data. The data processor is further configured to estimate a level of tissue ablation for the tissue region on basis of the in-plane strain, the out-of-plane motion, and a predetermined model, and the predetermined model at least reflecting a first causal relationship between the in-plane strain and the level of tissue ablation and a second causal relationship between the out-of-plane motion and the level of tissue ablation.

The term "strain" is also known as elastography. As well-understood in the art, the term "plane" refers to an imaging plane extending along the axial direction (i.e. the propagation direction of ultrasound signal), an in-plane strain refers to strain estimated within the imaging plane, and the out-of-plane motion refers to motion along a direction orthogonal to the imaging plane. The direction orthogonal to the imaging plane is a direction pointing outward the imaging plane, and thus is also called out-of-plane direction. For example, in the case that the imaging plane extends in the axial and azimuth directions, the out-of-plane direction is the elevational direction. The imaging plane can be the physical imaging plane along which the three-dimensional ultrasound echo data is acquired, or can be a virtual imaging plane which is synthesized from the three-dimensional ultrasound echo data.

In this way, the level of tissue ablation is estimated by utilizing its causal relationship with the in-plane strain but also its causal relationship with the out-of-plane motion. Thus, the estimation is expected to be more accurate and/or reliable. In particular, the in-plane strain reflects the stiffness of the tissue, and the out-of-plane motion reflects the stickiness of the tissue region to its surroundings. Contrary thereto, in conventional ultrasound elastography based ablation monitoring, only the causal relationship on the in-plane strain is utilized to estimate the level of tissue ablation, and the out-of-plane motion is merely regarded as artifacts which shall be compensated in deriving the in-plane strain. It is the present invention that firstly proposes to make use of the causal relationship between the out-of-plane motion and the level of tissue ablation to monitor ablation.

In an embodiment, the first causal relationship between the in-plane strain and the level of tissue ablation comprises a smaller strain being associated with a higher level of tissue ablation.

In an embodiment, the second relationship between the out-plane motion and the level of tissue ablation comprises a more out-of-plane motion being associated with a higher level of tissue ablation.

In some embodiments, the data processor is further configured to apply motion compensation when measuring the in-plane strain on basis of the measured out-of-plane motion. In other words, measuring the in-plane strain comprises applying motion compensation on basis of the measured out-of-plane motion. By means of motion compensation, the measured in-plane strain can be more accurate and/or reliable.

In some embodiments, the data processor is further configured to measure in-plane strain and out-of-plane motion regarding each of a plurality of tissue sub-regions within the tissue region, and to estimate the level of tissue ablation for each of the plurality of tissue sub-regions on basis of the in-plane strain and the out-of-plane motion regarding each of the plurality of tissue sub-regions, and the predetermined model. A tissue sub-region may comprise one or more adjacent spatial points within the tissue region.

In some embodiments, the data processor is further configured to derive at least one of a first parametric map indicative the measured in-plane strain regarding the plurality of tissue sub-regions and a second parametric map indicative the measured out-of-plane motion regarding the plurality of tissue sub-regions; and the data interface is further configured to output the derived at least one of the first and second parametric maps.

In an embodiment, the data interface can be communicatively connected to a user interface, and be configured to output the derived at least one of the first and second parametric maps to the user interface such that the derived at least one of the first and second parametric maps can be presented or displayed via the user interface. The user interface can be part of the apparatus or can be a separate apparatus.

In some embodiments, the data processor is further configured to determine at least a portion of the tissue region as an ablated zone on basis of the estimated levels of tissue ablation for the plurality of the tissue sub-region. For example, the ablated zone can be determined as comprising the portion of the tissue region where the estimated level of tissue ablation exceeds a predetermined level.

In some embodiments, the apparatus further comprises a user interface coupled to the data interface and configured to present the estimated level of tissue ablation for the tissue region. The estimated level of tissue ablation can be presented in various formats, including texture, graphical, or audio format. In some embodiments, a parameter map can be derived and presented, wherein each point of the parametric map represents the estimated level of tissue ablation of the corresponding spatial point in the tissue region.

In some embodiments, the data processor is further configured to measure the out-of-plane motion by calculating correlation coefficient(s) of the tissue region along the out-of-plane direction, and calculating the out-of-plane motion on basis of the calculated correlation coefficient(s) and a predetermined relationship between correlation coefficient(s) and displacement(s) along the out-of-plane direction.

In some embodiments, the predetermined relationship between correlation coefficient(s) and displacement(s) along the out-of-plane direction is dependent on an ultrasound probe configuration for acquiring the ultrasound echo data.

In an embodiment, the predetermined relationship can be embodied as a predetermined lookup table.

In an embodiment, there are a plurality of predetermined relationships between correlation coefficient(s) and displacement(s) along the out-of-plane direction, and each predetermined relationship corresponds to a specific ultrasound probe configuration.

In accordance with an embodiment of a second aspect of the present invention, there is proposed an ultrasound system for monitoring an ablation of a tissue region. The ultrasound system comprises an ultrasound probe for acquiring three-dimensional ultrasound echo data of the tissue region, and the apparatus for estimating a level of thermal ablation for a tissue region on basis of the three-dimensional ultrasound echo data received from the ultrasound probe. In some embodiments, the ultrasound probe may comprise a two dimensional matrix of transducer elements. In some other embodiments, the ultrasound probe may comprise a one dimensional linear or curved array. In case that the ultrasound probe is a 2D ultrasound probe, a sweeping scanning may be performed to acquire the three-dimensional data.

In accordance with an embodiment of a third aspect of the present invention, there is proposed a system for thermal ablation of a tissue region. The system comprises a thermal ablation apparatus for ablating the tissue region, and the ultrasound system for monitoring the thermal ablation of the tissue region as described above.

In accordance with an embodiment of a fourth aspect of the present invention, there is proposed a method of estimating a level of thermal ablation for a tissue region. The proposed method comprises: receiving the three-dimensional ultrasound echo data of the tissue region; measuring in-plane strain and out-of-plane motion regarding the tissue region on basis of the received ultrasound echo data; and estimating the level of tissue ablation for the tissue region on basis of the in-plane strain, the out-of-plane motion, and a predetermined model. The predetermined model at least reflects a first causal relationship between the in-plane strain and the level of tissue ablation and a second causal relationship between the out-of-plane motion and the level of tissue ablation. In some embodiment, the method further comprises a step of acquiring, via an ultrasound probe, the three-dimensional ultrasound echo data of the tissue region.

In accordance with an embodiment of a fifth aspect of the present invention, there is proposed a computer-readable medium comprising executable instructions, which when executed, cause a processor to perform any of the proposed methods.

Other objects and advantages of the present invention will become more apparent and can be easily understood with reference to the description made in combination with the accompanying drawings.

### DESCRIPTION OF THE DRAWINGS

The present invention will be described and explained hereinafter in more detail in combination with embodiments and with reference to the drawings, wherein:
FIG.1 illustrates an ultrasound system for monitoring a thermal ablation of a tissue region in accordance with some embodiments of the present invention;
FIG.2 illustrates a method of estimating a level of thermal ablation for a tissue region in accordance with some embodiments of the present invention; and
FIG.3 illustrates a schematic diagram of estimating a level of thermal ablation for a tissue region in accordance with some embodiments of the present invention.

The same reference signs in the figures indicate similar or corresponding features and/or functionalities.

### DETAILED DESCRIPTION

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn to scale for illustrative purposes.

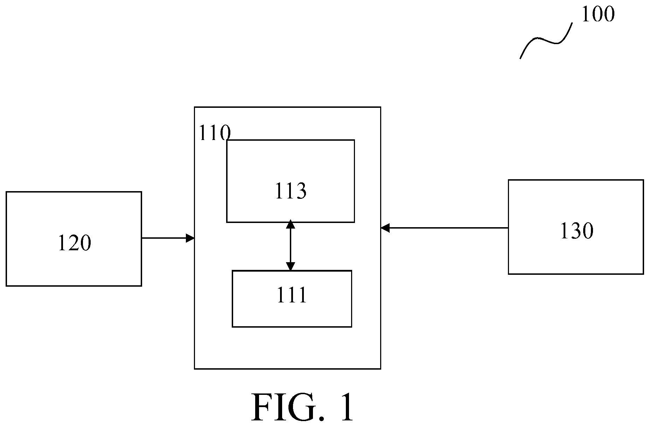

FIG.1 illustrates an ultrasound system 100 for monitoring a thermal ablation of a tissue region in accordance with some embodiments of the present invention.

The ultrasound system 100 comprises an apparatus 110 for estimating a level of thermal ablation for a tissue region, an ultrasound probe 120, and a user interface 130. The apparatus 110 is communicatively connected to the ultrasound probe 120 and the user interface 130 in various ways, including wired or wireless connections, local or remote connections, etc. The user interface 130 may optionally be communicatively connected to the ultrasound probe 120 as well. The apparatus 110, the ultrasound probe 120, and the user interface 130 can be integrated into a single device, or can be physically separated from one or another. Each of the apparatus 110, the ultrasound probe 120 and the user interface 130 can comprise one or more devices.

The ultrasound probe 120 can be any kind of device that comprises a plurality of transducers capable of transmitting and/or receiving ultrasound signals. Typically, the ultrasound probe 120 is configured to both transmit ultrasound signals toward a region of interest and receive echoes of the transmitted ultrasound signals from the region of interest. In accordance with some embodiments of the present invention, the ultrasound probe 120 is configured to acquire three dimensional ultrasound data of the tissue region. In some examples, the ultrasound probe 120 may comprise a matrix of transducers capable of scanning a volume. In some other examples, the ultrasound probe 120 may comprise an array of transducers capable of directly scanning a plane. The array of transducers is repositioned and/or re-oriented to scanning multiple, different planes, either automatically or manually, and the two dimensional ultrasound data of multiple planes can be assembled into three dimensional ultrasound data of a volume of interest. For example, the ultrasound probe 120 can be a mechanical three-dimensional ultrasound probe.

Furthermore, the ultrasound probe 120 is configured to acquire three-dimensional ultrasound echo data which is sufficient for deriving in-plane strain and out-of-plane motion. In some embodiments, the three-dimensional ultrasound echo data comprises a temporal sequence of three-dimensional ultrasound data frames.

As illustrated in FIG. 1, the apparatus 110 comprises a data interface 111 and a data processor 113. The data interface 111 is configured to receive the three-dimensional ultrasound echo data of the tissue region. Typically, ultrasound echo signals received by the transducers will be beamformed, the beamformed signals are processed by various signal processing, such as bandpass filtering, decimation, I and Q component separation, harmonic signal separation, speckle reduction, signal compounding, and/or noise elimination, and the processed signals can be further processed to obtain B-mode data, Doppler data, strain data, motion data, etc. Most of these signal/data processing can be performed by either the ultrasound probe 120 or the apparatus 110. In some embodiments, the ultrasound probe 120 may be configured to provide just raw ultrasound signals such as radio-frequency ultrasound signals, or may be configured to provide B-mode data, or may be configured to provide strain data or motion data. Correspondingly, the ultrasound echo data received by the data interface 111 can be in various format.

The data processor 113 is configured to measure in-plane strain and out-of-plane motion regarding the tissue region on basis of the received ultrasound echo data. The in-plane strain and out-of-plane motion can be measured in any kind of existing approaches or approaches developed in future. The tissue region can be two dimensional or three dimensional. In some embodiments, the in-plane strain and out-of-plane motion are measured for each point in the tissue region. More generally, in some embodiments, the tissue region comprises a plurality of sub-region, each sub-region may comprise one or more adjacent points, and the in-plane strain and out-of-plane motion are measured for each tissue sub-region.

In accordance with some embodiments of the present invention, the data processor 113 is configured to estimate the level of tissue ablation for the tissue region on basis of the in-plane strain, the out-of-plane motion, and a predetermined model. The predetermined model at least reflects a first causal relationship between the in-plane strain and the level of tissue ablation and a second causal relationship between the out-of-plane motion and the level of tissue ablation.

The predetermined model is normally trained off-line on basis of a large amount data, and can be optionally upgraded during the use. The predetermined model can be generated by various techniques. The predetermined model can be traditional deterministic models with deterministic features and fixed or adaptive thresholds. The predetermined model can be generated by more advanced techniques, such as deep learning.

In some embodiments, the measured out-of-plane motion is further used to apply motion compensation to the in-plane strain so as to get a more reliable measurement of the in-plane strain. In some embodiments, the motion compensation is performed by the predetermined model. In some other embodiments, the in-plane strain is motion compensated prior to be input to the predetermined model, and the motion-compensated in-plane strain is input to the predetermined model.

Referring to FIG. 1, the user interface 130 is communicatively connected to the apparatus 120. The user interface 130 is configured to present the estimated level of tissue ablation for the tissue region. The presentation of the estimated level of tissue ablation can be various in terms of content and/or format. In some embodiments, the estimated level of tissue ablation can be formatted as a parametric map. For example, the parametric map can be colored. The parametric map can be displayed via the user interface. For example, the parametric map can be displayed as an overlay over the B-mode image of the tissue region. The parametric map can be either two dimensional for a selected plane or three dimensional for a selected volume or the whole volume of tissue region. In some embodiments, the data processor 113 is further configured to determine at least a portion of the tissue region as an ablated zone on basis of the estimated level of tissue ablation. For example, the ablated zone comprises portions of the tissue region where the estimated level of tissue ablation reaches a predetermined threshold. The determined ablated zone can be displayed via the user interface. Additionally or alternatively, the determined ablated zone can be compared with a target ablation zone. Typically, the thermal ablation would be regarded as being completed when the target ablation zone is determined as ablated within a predetermined tolerance. For example, an indicator can be generated for indicating the progress of the ablation, and the indicator can be presented to the user in visual and/or audio ways.

In some embodiments, the data processor 113 is further configured to derive at least one of a first parametric map indicative the measured in-plane strain regarding the plurality of tissue sub-regions and a second parametric map indicative the measured out-of-plane motion regarding the plurality of tissue sub-regions, and the user interface 130 is further configured to present the at least one of the first and second parametric maps. The

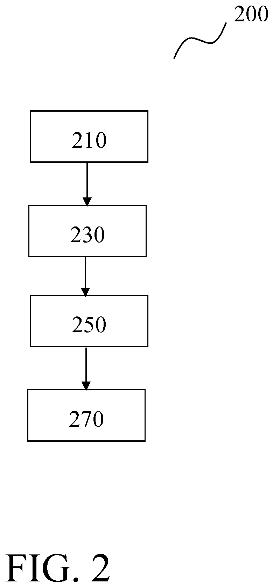

FIG.2 illustrates a method 200 of estimating a level of thermal ablation for a tissue region in accordance with some embodiments of the present invention. In step 210, the three-dimensional ultrasound echo data of the tissue region is received via the data interface. In step 230, out-of-plane motion regarding the tissue region is measured on basis of the received ultrasound echo data. In step 250, in-plane strain regarding the tissue region on basis of the received ultrasound echo data. The step 230 of measuring the out-of-plane motion and the step 250 of measuring the in-plane strain can be performed simultaneously or sequentially, and the order of steps 230 and 250 can be altered. In step 270, the level of tissue ablation for the tissue region is estimated on basis of the in-plane strain, the out-of-plane motion, and a predetermined model, the predetermined model at least reflecting a first causal relationship between the in-plane strain and the level of tissue ablation and a second causal relationship between the out-of-plane motion and the level of tissue ablation.

In some embodiments, the method 200 may further comprise a step of acquiring, via an ultrasound probe, the three-dimensional ultrasound echo data of the tissue region. In some embodiments, the method 200 may further comprise a step of presenting the estimated level of tissue ablation and/or other output derived from the estimated level tissue ablation, such as the determined ablated zone, via user interface.

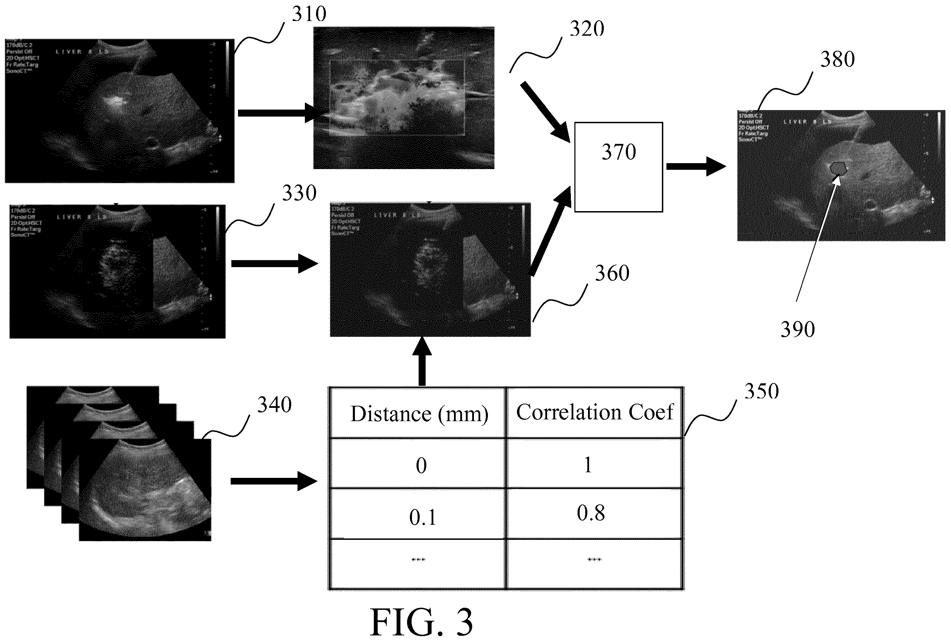

FIG.3 illustrates a schematic diagram of estimating a level of thermal ablation for a tissue region in accordance with some embodiments of the present invention.

Referring to FIG. 3, block 310 illustrates that ultrasound data, such as a temporal sequence of three dimensional ultrasound data frames, is acquired after ablation. Block 320 illustrates that ultrasound elastography is generated using the acquired ultrasound data. Ultrasound elastography is known as an ultrasound imaging modality that maps the elastic properties (such as in-plane strain) of soft tissue. For example, a strain map is displayed as an overlay to the B-mode image. Block 360 illustrates that an elevational motion map indicative the motion along the elevational direction, is generated using the acquired ultrasound data. Block 370 illustrates the predetermined model by means of which the level of tissue ablation can be estimated on basis of the strain map and the elevational motion map. Block 380 illustrates the estimated level of thermal ablation. For example, an ablated zone 390 is delineated on basis of the estimated level of thermal ablation.

In accordance with an embodiment of the present invention, the data processor is further configured to measure the out-of-plane motion by calculating correlation coefficients of the tissue region along the out-of-plane direction. Due to nonlinear speckle decorrelation, the correlation coefficients shall be calibrated so as to be transformed to out-of-plane motion. In some embodiments, the data processor is further configured to calculate the out-of-plane motion on basis of the calculated correlation coefficients and a predetermined relationship between correlation coefficients and displacements along the out-of-plane direction. A lower correlation coefficient of a tissue region normally correspond to a larger displacement of the tissue region. The predetermined relationship between correlation coefficient(s) and displacement(s) along the out-of-plane direction is dependent on an ultrasound probe configuration for acquiring the ultrasound echo data. In some embodiments, the predetermined relationship can be generated using pre-acquired phantom data with known elevational distance. Optionally, a lookup table can be generated to represent the predetermined relationship.

Referring to FIG. 3, Block 330 illustrates that elevational correlation coefficient map is calculated using the acquired ultrasound data. Block 340 illustrates that ultrasound data of a phantom with known elevation distance is pre-acquired with an ultrasound probe having the same configuration as the one for acquiring the ultrasound echo data from the tissue region on which thermal ablation is applied. For example, the ultrasound data of the phantom comprises ultrasound data frames of a plurality of scanning plane separated by known distance(s). Block 350 illustrates the lookup table. For example, each entry of the lookup table comprises a coefficient value and a corresponding distance value.

The technique processes described herein may be implemented by various means. For example, these techniques may be implemented in hardware, software, or a combination thereof. For a hardware implementation, the technical processes may be implemented within one or more application specific integrated circuits (ASICs), digital signal processors (DSPs), digital signal processing devices (DSPDs), programmable logic devices (PLDs), field programmable gate arrays (FPGAs), processors, controllers, microcontrollers, microprocessors, other electronic units designed to perform the functions described herein, or a combination thereof. With software, implementation can be through modules (e.g., procedures, functions, and so on) that perform the functions described herein. The software codes may be stored in a volatile or non-volatile storage medium and executed by the processors.

Moreover, aspects of the claimed subject matter may be implemented as a method, apparatus, system, or article of manufacture using standard programming and/or engineering techniques to produce software, firmware, hardware, or any combination thereof to control a computer or computing components to implement various aspects of the claimed subject matter. The term "article of manufacture" as used herein is intended to encompass a computer program accessible from any computer-readable device, carrier, or media. For example, computer readable media can include but are not limited to magnetic storage devices (e.g., hard disk, floppy disk, magnetic strips...), optical disks (e.g., compact disk (CD), digital versatile disk (DVD)...), smart cards, and flash memory devices (e.g., card, stick, key drive...).

As used in this application, the terms "data interface", "processor" such as data processor, are intended to refer to a general-purpose processor, a specific-purpose processor, a computer processor, or a computer-related entity, either hardware, a combination of hardware and software, software, or software in execution. For example, a component may be, but is not limited to, a process running on a processor, a processor, an object, an executable, a thread of execution, a program, and/or a computer. By way of illustration, both an application running on a server and the server can be a component. One or more components may reside within a process and/or thread of execution and a component may be localized on one computer and/or distributed among two or more computers.

What has been described above includes examples of one or more embodiments. It is, of course, not possible to describe every conceivable combination of components or methodologies for the purpose of describing the aforementioned embodiments, but one of ordinary skill in the art may recognize that many further combinations and permutations of various embodiments are possible. Accordingly, the described embodiments are intended to embrace all such alterations, modifications and variations that fall within the scope of the appended claims. Furthermore, to the extent that the term "includes" is used in either the detailed description or the claims, such term is intended to be inclusive in a manner similar to the term "comprising" as "comprising" is interpreted when employed as a transitional word in a claim.

## Claims

1. An apparatus (110) for estimating a level of thermal ablation for a tissue region on basis of three-dimensional ultrasound echo data of the tissue region, comprising:
a data interface (111) configured to receive the three-dimensional ultrasound echo data of the tissue region; and
**characterized by**
a data processor (113) configured to measure in-plane strain and out-of-plane motion regarding the tissue region on basis of the received ultrasound echo data;
wherein the data processor (113) is further configured to estimate a level of tissue ablation for the tissue region on basis of the in-plane strain, the out-of-plane motion, and a predetermined model, and the predetermined model at least reflecting a first causal relationship between the in-plane strain and the level of tissue ablation and a second causal relationship between the out-of-plane motion and the level of tissue ablation.

2. The apparatus of Claim 1, wherein the second relationship between the out-plane motion and the level of tissue ablation comprises a more out-of-plane motion being associated with a higher level of tissue ablation.

3. The apparatus of Claim 1, wherein the data processor is further configured to apply motion compensation when measuring the in-plane strain on basis of the measured out-of-plane motion.

4. The apparatus of Claim 1, wherein the data processor is further configured to measure in-plane strain and out-of-plane motion regarding each of a plurality of tissue sub-regions within the tissue region, and to estimate the level of tissue ablation for each of the plurality of tissue sub-regions on basis of the in-plane strain and the out-of-plane motion regarding each of the plurality of tissue sub-regions, and the predetermined model.

5. The apparatus of Claim 4, wherein;
the data processor is further configured to derive at least one of a first parametric map indicative the measured in-plane strain regarding the plurality of tissue sub-regions and a second parametric map indicative the measured out-of-plane motion regarding the plurality of tissue sub-regions; and
the data interface is further configured to output the derived at least one of the first and second parametric maps.

6. The apparatus of Claim 4, wherein the data processor is further configured to determine at least a portion of the tissue region as an ablated zone on basis of the estimated level of tissue ablation for the plurality of the tissue sub-region.

7. The apparatus of Claim 1, further comprising a user interface (130) coupled to the data interface and configured to present the estimated level of tissue ablation for the tissue region.

8. The apparatus of Claim 1, wherein the data processor is further configured to measure the out-of-plane motion by calculating correlation coefficient(s) of the tissue region along an out-of-plane direction, and calculating the out-of-plane motion on basis of the calculated correlation coefficient(s) and a predetermined relationship between correlation coefficient(s) and displacement(s) along the out-of-plane direction.

9. The apparatus of Claim 8, wherein the predetermined relationship between correlation coefficient(s) and displacement(s) along the out-of-plane direction is dependent on an ultrasound probe configuration for acquiring the ultrasound echo data.

10. An ultrasound system (100) for monitoring a thermal ablation of a tissue region, comprising:
an ultrasound probe (120) for acquiring ultrasound echo data of the tissue region; and
an apparatus of Claim 1 coupled to the ultrasound probe to receive the acquired ultrasound echo data of the tissue region.

11. The ultrasound system of Claim 10, wherein the ultrasound probe comprises a two-dimensional matrix of transducer elements.

12. A system for thermal ablation of a tissue region, comprising:
a thermal ablation apparatus for ablating the tissue region;
an ultrasound system (100) for monitoring the thermal ablation of the tissue region of Claim 10.

13. A method (200) of estimating a level of thermal ablation for a tissue region, the method comprising:
receiving (210) three-dimensional ultrasound echo data of the tissue region;
**characterized by** comprising:
measuring (230) out-of-plane motion regarding the tissue region on basis of the received ultrasound echo data;
measuring (250) in-plane strain regarding the tissue region on basis of the received ultrasound echo data; and
estimating (270) a level of tissue ablation for the tissue region on basis of the in-plane strain, the out-of-plane motion, and a predetermined model, the predetermined model at least reflecting a first causal relationship between the in-plane strain and the level of tissue ablation and a second causal relationship between the out-of-plane motion and the level of tissue ablation.

14. The method of Claim 13, further comprising a step of acquiring, via an ultrasound probe, the three-dimensional ultrasound echo data of the tissue region.

15. A computer-readable medium comprising executable instructions, which when executed, cause a processor to perform the method of claim 13.

## Patentansprüche

1. Vorrichtung (110) zum Schätzen eines Grads thermischer Ablation für einen Gewebebereich basierend auf dreidimensionalen Ultraschallechodaten des Gewebebereichs, umfassend:
eine Datenschnittstelle (111), die konfiguriert ist, um die dreidimensionalen Ultraschallechodaten des Gewebebereichs zu empfangen; und
**gekennzeichnet durch**
einen Datenprozessor (113), der konfiguriert ist, um basierend auf den empfangenen Ultraschallechodaten eine Dehnung in der Ebene und eine Bewegung außerhalb der Ebene in Bezug auf den Gewebebereich zu messen;
wobei der Datenprozessor (113) ferner konfiguriert ist, um einen Grad von Gewebeablation für den Gewebebereich basierend auf der Dehnung in der Ebene, der Bewegung außerhalb der Ebene und einem vorbestimmten Modell zu schätzt, und wobei das vorbestimmte Modell mindestens eine erste kausale Beziehung zwischen der Dehnung in der Ebene und dem Grad von Gewebeablation und eine zweite kausale Beziehung zwischen der Bewegung außerhalb der Ebene und dem Grad von Gewebeablation widerspiegelt.

2. Vorrichtung nach Anspruch 1, wobei die zweite Beziehung zwischen der Bewegung außerhalb der Ebene und dem Grad von Gewebeablation darin besteht, dass eine größere Bewegung außerhalb der Ebene mit einem höheren Grad von Gewebeablation assoziiert ist.

3. Vorrichtung nach Anspruch 1, wobei der Datenprozessor ferner konfiguriert ist, um beim Messen der Dehnung in der Ebene eine Bewegungskompensation basierend auf der gemessenen Bewegung außerhalb der Ebene anzuwenden.

4. Vorrichtung nach Anspruch 1, wobei der Datenprozessor ferner konfiguriert ist, um eine Dehnung in der Ebene und eine Bewegung außerhalb der Ebene in Bezug auf jeden aus einer Vielzahl von Gewebeunterbereichen innerhalb des Gewebebereichs zu messen und den Grad von Gewebeablation für jeden aus der Vielzahl von Gewebeunterbereichen basierend auf der Dehnung in der Ebene und der Bewegung außerhalb der Ebene in Bezug auf jeden aus der Vielzahl von Gewebeunterbereichen und das vorbestimmte Modell zu schätzen.

5. Vorrichtung nach Anspruch 4, wobei der Datenprozessor ferner konfiguriert ist, um mindestens eine erste parametrische Karte, die die gemessene Dehnung in der Ebene in Bezug auf die mehreren Gewebeunterbereichen angibt, und eine zweite parametrische Karte, die die gemessene Bewegung außerhalb der Ebene in Bezug auf die mehreren Gewebeunterbereichen angibt, abzuleiten; und die Datenschnittstelle ferner konfiguriert, um die abgeleitete erste und/oder zweite parametrische Karte auszugeben.

6. Vorrichtung nach Anspruch 4, wobei der Datenprozessor ferner konfiguriert ist, um mindestens einen Teil des Gewebereichs basierend auf dem geschätzten Grad von Gewebeablation für die Vielzahl der Gewebeunterbereiche als eine ablatierte Zone zu bestimmen.

7. Vorrichtung nach Anspruch 1, ferner umfassend eine Benutzerschnittstelle (130), die mit der Datenschnittstelle gekoppelt und konfiguriert ist, um den geschätzten Grad von Gewebeablation für den Gewebebereich darzustellen.

8. Vorrichtung nach Anspruch 1, wobei der Datenprozessor ferner konfiguriert ist, um die Bewegung außerhalb der Ebene zu messen, indem er Korrelationskoeffizient(en) des Gewebebereichs entlang einer Richtung außerhalb der Ebene berechnet und die Bewegung außerhalb der Ebene basierend auf dem/den berechneten Korrelationskoeffizient(en) und einer vorbestimmten Beziehung zwischen Korrelationskoeffizient(en) und Verschiebung(en) entlang der Richtung außerhalb der Ebene berechnet.

9. Vorrichtung nach Anspruch 8, wobei die vorbestimmte Beziehung zwischen Korrelationskoeffizient(en) und Verschiebung(en) entlang der Richtung außerhalb der Ebene von einer Ultraschallsondenkonfiguration zum Erfassen der Ultraschallechodaten abhängt.

10. Ultraschallsystem (100) zum Überwachen einer thermischen Ablation eines Gewebebereichs,
umfassend:
eine Ultraschallsonde (120) zum Erfassen von Ultraschallechodaten des Gewebebereichs; und eine Vorrichtung nach Anspruch 1, die mit der Ultraschallsonde gekoppelt ist, um die erfassten Ultraschallechodaten des Gewebebereichs zu empfangen.

11. Ultraschallsystem nach Anspruch 10, wobei die Ultraschallsonde eine zweidimensionale Matrix von Wandlerelementen umfasst.

12. System zur thermischen Ablation eines Gewebebereichs, umfassend:
eine thermische Ablationsvorrichtung zur Ablation des Gewebebereichs;
ein Ultraschallsystem (100) zum Überwachen der thermischen Ablation des Gewebebereichs nach Anspruch 10.

13. Verfahren (200) zum Schätzen eines Grads thermischer Ablation für einen Gewebebereich, das Verfahren umfassend:
empfangen (210) dreidimensionaler Ultraschallechodaten des Gewebebereichs;
**dadurch gekennzeichnet, dass** es Folgendes umfasst:
Messen (230) der Bewegung außerhalb der Ebene in Bezug auf den Gewebebereich basierend auf den empfangenen Ultraschallechodaten;
Messen (250) der Dehnung in der Ebene in Bezug auf den Gewebebereich basierend auf den empfangenen Ultraschallechodaten; und
Schätzen (270) eines Grads von Gewebeablation für den Gewebebereich basierend auf der Dehnung in der Ebene, der Bewegung außerhalb der Ebene und einem vorbestimmten Modell, wobei das vorbestimmte Modell mindestens eine erste kausale Beziehung zwischen der Dehnung in der Ebene und dem Grad von Gewebeablation und eine zweite kausale Beziehung zwischen der Bewegung außerhalb der Ebene und dem Grad von Gewebeablation widerspiegelt.

14. Verfahren nach Anspruch 13, ferner umfassend einen Schritt eines Erfassens der dreidimensionalen Ultraschallechodaten des Gewebebereichs über eine Ultraschallsonde.

15. Computerlesbares Medium, umfassend ausführbare Anweisungen, die, wenn sie ausgeführt werden, einen Prozessor veranlassen, das Verfahren nach Anspruch 13 auszuführen.

## Revendications

1. Un appareil (110) pour estimer un niveau d'ablation thermique pour une région tissulaire à partir de données d'échographie tridimensionnelle de la région tissulaire, comprenant:
une interface de données (111) configurée pour recevoir des données d'échographie de la région du tissu; et
est **caractérisé par**:
un processeur de données (113) configuré pour mesurer une déformation dans le plan et un mouvement hors du plan en ce qui concerne la région tissulaire à partir de données d'échographie de la région tissulaire;
où le processeur de données (113) est également configuré pour estimer un niveau d'ablation thermique pour la région tissulaire à partir de la déformation dans le plan, le mouvement hors plan, et un modèle prédéterminé, et le modèle prédéterminé reflétant au moins une première relation entre la déformation dans le plan et le niveau de l'ablation du tissu et une seconde relation de cause à effet entre le mouvement hors plan et le niveau de l'ablation du tissu.

2. L'appareil selon la revendication 1, où la seconde relation entre le mouvement hors plan et le niveau du tissu d'ablation comprend un mouvement plus hors plan étant associé avec un niveau supérieur de l'ablation de tissu.

3. L'appareil de la revendication 1, où le processeur de données est également configuré pour appliquer la compensation du mouvement lors de la mesure de la déformation dans le plan à partir d'un mouvement hors plan mesuré.

4. L'appareil de la revendication 1, où le processeur de données est également configuré pour mesurer la déformation dans le plan et un mouvement hors plan concernant chacune une pluralité de sous-régions tissulaires dans la région du tissu, et pour estimer le niveau de l'ablation du tissu pour chacune des pluralités des tissus des sous-régions à partir de la déformation dans le plan et le mouvement hors plan par rapport à chacune des pluralités de sous-régions de tissus, et le modèle prédéterminé.

5. L'appareil de la revendication 4, où le processeur de données est également configuré pour obtenir au moins une première carte paramétrique indiquant la déformation dans le plan mesuré en ce qui concerne la pluralité de sous-régions tissulaires et une deuxième carte paramétrique indiquant un mouvement hors plan mesuré en ce qui concerne la pluralité de sous-régions tissulaires; et l'interface de donnée est également configurée pour obtenir le résultat dérivé au moins des premières et secondes cartes paramétriques.

6. L'appareil de la revendication 4, où le processeur de données est également configuré pour déterminer au moins une partie de la région tissulaire en tant que zone d'ablation à partir du niveau estimé de l'ablation de tissu pour la pluralité de la sous-région tissulaire.

7. L'appareil de la revendication 1 comprend également une interface utilisateur (130) couplée à l'interface de données et configurée pour présenter le niveau estimé de l'ablation tissulaire pour la région tissulaire.

8. L'appareil de la revendication 1, où le processeur de données est également configuré pour mesurer un mouvement hors plan en calculant des coefficient(s) de corrélation de la région tissulaire le long d'une direction hors plan, et calculer le mouvement hors plan à partir des coefficient(s) de corrélation calculés et une relation prédéterminée entre les coefficient(s) de corrélation et le déplacement(s) le long d'une direction hors plan.

9. L'appareil selon la revendication 8, où la relation prédéterminée entre la corrélation de coefficient(s) et le déplacement(s) le long d'une direction hors plan est dépendante d'une configuration d'une sonde échographique pour obtenir des données échographiques.

10. Un système d'échographie (100) pour surveiller une ablation thermique d'une région tissulaire comprend: une sonde d'imagerie (120) pour l'acquisition des données d'échographie de la région tissulaire; et un appareil de la revendication 1 couplé à la sonde d'échographie pour recevoir les données d'échographie obtenues de la région tissulaire.

11. Le système d'échographie de la revendication 10, où la sonde d'échographie comprend un réseau bidimensionnel d'éléments transducteurs.

12. Un système de l'ablation thermique de la région tissulaire, comprend:
un appareil d'ablation pour l'ablation de la région tissulaire;
un système d'échographie (100) pour surveiller l'ablation thermique de la région tissulaire de la revendication 10.

13. Une méthode (200) d'estimation d'un niveau d'ablation thermique pour une région tissulaire, la méthode comprend:
la réception (210) des données échographiques tridimensionnelles de la région tissulaire;
caractérisé en comprenant:
la mesure (230) d'un mouvement hors plan en ce qui concerne la région tissulaire à partir des données d'échographie reçues;
la mesure (250) de la déformation dans le plan en ce qui concerne la région tissulaire à partir des données d'échographie reçues; et
l'estimation (270) d'un niveau d'ablation du tissu pour la région tissulaire à partir de la déformation dans le plan, le mouvement hors plan, et le modèle prédéterminé, le modèle prédéterminé reflète au moins une première relation de cause à effet entre la déformation dans le plan et le niveau de l'ablation tissulaire et une seconde relation de cause à effet entre le mouvement hors plan et le niveau de l'ablation tissulaire.

14. La méthode selon la revendication 13, comprend également une étape de l'obtention à travers une sonde d'échographie, les données d'échographie tridimensionnelles de la région tissulaire.

15. Un support lisible par ordinateur comprend des instructions exécutables, qui lorsqu'elles sont exécutées, font en sorte que le processeur exécute la méthode de la revendication 13.
